# EUROPEAN PATENT APPLICATION

(11) **EP 2 894 477 A1**
(43) Date of publication of application: **15.07.2015**
(21) Application number: 13835821.3
(22) Date of filing: 03.09.2013
(51) Int. Cl.: G01N 33/68, G01N 33/53, G01N 33/574

(54) **METHOD FOR DETERMINING BREAST CANCER**

(30) Priority: 05.09.2012 JP 2012194804
(71) Applicant: Wako Pure Chemical Industries, Ltd., Osaka-shi Osaka 540-8605 (JP); Osaka University, Suita-shi, Osaka 565-0871 (JP)
(72) Inventor: MATSUURA Shuji, Suita-shi Osaka 565-0871 (JP); MATSUURA Nariaki, Suita-shi Osaka 565-0871 (JP); KURONO Sadamu, Osaka 559-0012 (JP); KANEKO Yuka, Osaka 559-0012 (JP)
(74) Representative: Winter, Brandl, Fürniss, Hübner, Röss, Kaiser, Polte - Partnerschaft mbB
(86) International application number: PCT/JP2013/073594
(87) International publication number: WO 2014/038524

(57) **Abstract**

The present invention relates to: a breast cancer marker which is selected from the group consisting of carboxypeptidase N subunit 2, extracellular matrix protein 1, serum amyloid P component, nebulin, complement component C8 α chain, apolipoprotein L1, flavin reductase, catalase, carbonic anhydrase 2, apolipoprotein C-I, nuclear pore glycoprotein 210, superoxide dismutase [Cu-Zn], bisphosphoglycerate mutase, carbonic anhydrase 1 and peroxiredoxin-2; a method for determining breast cancer, which comprises detecting the breast cancer marker in a sample and determining breast cancer on the basis of the results of the detection; and a kit for use in the method.

## Description

### TECHNICAL FIELD

The present invention relates to a novel breast cancer marker, and a method for determining (diagnosing or testing) breast cancer comprising detecting the marker and determining on the basis of the results, and a kit to be used for said determination.

### BACKGROUND ART

At present, as a method for diagnosing breast cancer, the diagnosis based on the detection of breast cancer marker in a sample such as body fluid or blood, or the diagnosis by mammography is carried out.

In the method for diagnosing breast cancer by detecting a breast cancer marker, using a carcinoembryonic antigen (CEA) as a marker of breast cancer, the amount of the marker in a sample is measured by using a diagnostic kit and the like, and the breast cancer is diagnosed on the basis of the results of the measurement. However, since accuracy indicating breast cancer specificity is poor, this method has not been established as a definitive diagnosis of breast cancer for practical use.

In addition, JP-A-2002-131322 (Patent Literature 1) discloses a method of identifying a patient having breast cancer or breast pre-cancer using a lactiferous duct fluid from a subject as a sample and detecting the components of the lactiferous duct fluid. However, although said literature describes many marker candidates including extracellular matrix protein or apolipoprotein, it only lists the marker candidates, and the component analyses of the lactiferous duct fluid of the breast cancer patients have not been carried out. Therefore, from the disclosure of Patent Literature 1, a large number of markers listed therein are unknown whether it is really useful as breast cancer markers.

In addition, JP- A - 2008-502891 (Patent Literature 2) discloses that a tissue piece from breast cancer patients was analyzed by LC-ESI-MS/MS (liquid chromatography□electrospray ionization□tandem mass spectrometry), and PDX1 (peroxiredoxin-1) was selected as a candidate of breast cancer marker, and that the PDX1 was detected in the serum of breast cancer patients. In addition, there is a description suggesting that the breast cancer may be diagnosed by combinational use of PDX1 with other markers. However, the evidence indicating that these markers are specific for breast cancer, for example, the measurement results of these markers in a sample from non-breast cancer subjects and the like have not been disclosed. Therefore, PDX1 has not been confirmed on whether it is useful as a marker for breast cancer, and whether the combination with other markers can be used in the diagnosis of breast cancer.

In addition, in the method for diagnosis of breast cancer using mammography, mental and physical distress suffered by medical examinee of the test (subject) is not small. In addition, because imaging of young people with high breast density is difficult, it has also not been accepted as a breast cancer diagnosis technology at a high level of accuracy.

Beyond that, the investigations on the search for biomarkers (proteins/peptides, lipids, sugar chains, etc.) are actively carried out in recent years, and comprehensive analysis study of proteomics and the like using a variety of body fluids particularly in connection with the disease have been carried out actively. These analyses can be carried out with high sensitivity and accurate analysis of trace samples obtained from a living organism. In this regard, studies on the breast cancer marker conducted using proteomics is introduced by F. Mannello et al. in Expert Rev. Proteomics 6, 43-60 (2009) (Non-Patent Literature 1). However, as for the breast cancer marker, the cancer marker with high accuracy in breast cancer diagnosis has not been found yet.

### CITATION LIST

### Patent Literature

Patent Literature 1: JP-A-2002-131322;
Patent Literature 2: JP-A-2008-502891.

### Non-Patent Literature

Non-Patent Literature 1: F. Mannello et al. Expert Rev. Proteomics 6, 43-60 (2009).

### SUMMARY OF INVENTION

### Technical Problem

Because there is a problem such as described above in the conventional method for diagnosing breast cancer, screening rate of breast cancer has not been improved, and the diagnosis of breast cancer and early-stage breast cancer is delayed, as a result, this has caused determent on the reduction of prevalence rate and mortality rate of breast cancer, and has become a major social problem. In order to solve these problems, the development of a new breast cancer diagnostic technique using a minimally-invasive and highly accurate breast cancer marker protein as an indicator of breast cancer diagnosis has been awaited.

An object of the present invention is to provide a novel breast cancer marker useful for detecting breast cancer, and a novel method for determining breast cancer with a high accuracy by detecting said breast cancer marker.

### Solution to Problem

The present invention was made for the purpose of solving the above-described problems, and made up of the following constructions,
(1) A breast cancer marker selected from the group consisting of carboxypeptidase N subunit 2, extracellular matrix protein 1, serum amyloid P-component, nebulin, complement component C8 α chain, apolipoprotein L1, flavin reductase, catalase, carbonic anhydrase 2, apolipoprotein C-I, nuclear pore glycoprotein 210, superoxide dismutase [Cu-Zn], bisphosphoglycerate mutase, carbonic anhydrase 1, and peroxiredoxin-2.
(2) A method for determining breast cancer, comprising: detecting one or more breast cancer markers selected optionally from the group consisting of carboxypeptidase N subunit 2, extracellular matrix protein 1, serum amyloid P-component, nebulin, complement component C8 α chain, apolipoprotein L1, flavin reductase, catalase, carbonic anhydrase 2, apolipoprotein C-I, nuclear pore glycoprotein 210, superoxide dismutase [Cu-Zn], bisphosphoglycerate mutase, carbonic anhydrase 1, and peroxiredoxin-2 in a sample, and determining breast cancer on the basis of the results of the detection.
(3) A kit for detecting a breast cancer marker to determine breast cancer comprising a reagent for detecting breast cancer marker selected from the group consisting of carboxypeptidase N subunit 2, extracellular matrix protein 1, serum amyloid P-component, nebulin, complement component C8 α chain, apolipoprotein L1, flavin reductase, catalase, carbonic anhydrase 2, apolipoprotein C-I, nuclear pore glycoprotein 210, superoxide dismutase [Cu-Zn], bisphosphoglycerate mutase, carbonic anhydrase 1, and peroxiredoxin-2.

That is, in the course of intensive investigation to establish a breast cancer marker with high accuracy for diagnosis of breast cancer which solved the above described problems and to establish a method of determining breast cancer using the same breast cancer marker, by using the nipple discharge (ND) as a sample, from which the information of the breast duct of a breast cancer onset site can be obtained directly, the present inventors have analyzed the components of the nipple discharge containing a large number of components with the application of proteomics by nano LC-ESI-MS/MS of the most recent two-dimensional liquid chromatography (LC) / mass spectrometry (MS) system. Then, using the nipple discharge derived from breast cancer patient and the nipple discharge derived from non-breast cancer subject as a sample, and by comparing the results of the nano LC-ESI-MS/MS, new markers to be presumably promising as breast cancer markers were selected. Then, the present inventors have found that if these markers are used as an indicator, it is possible to determine the breast cancer with good accuracy, and have thus completed the present invention.

### Advantageous Effects of Invention

Determination method using the breast cancer marker(s) of the present invention allows the determination (diagnosis, test) of breast cancer with higher accuracy. In addition, the breast cancer marker(s) of the present invention is found, for example, in the nipple discharge, therefore, it is possible to determine (diagnose, test) the breast cancer non-invasively and easily.

### BRIEF DESCRIPTION OF DRAWINGS

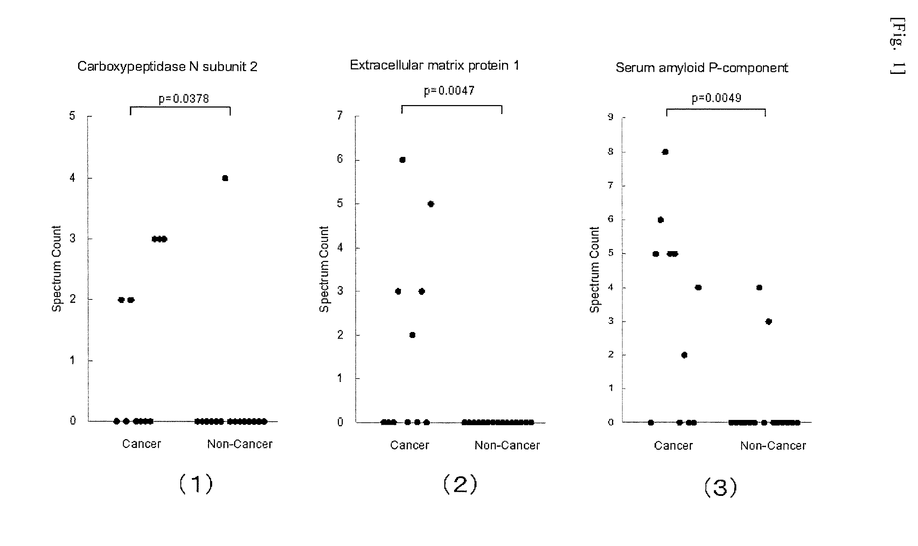

Fig. 1 shows the results of detection of each protein component in a sample derived from breast cancer patients and non-breast cancer subjects obtained in Example 1 and the statistical results obtained based on this result, wherein represents the results of measurement of (1) carboxypeptidase N subunit 2, (2) extracellular matrix protein 1, and (3) serum amyloid P-component.

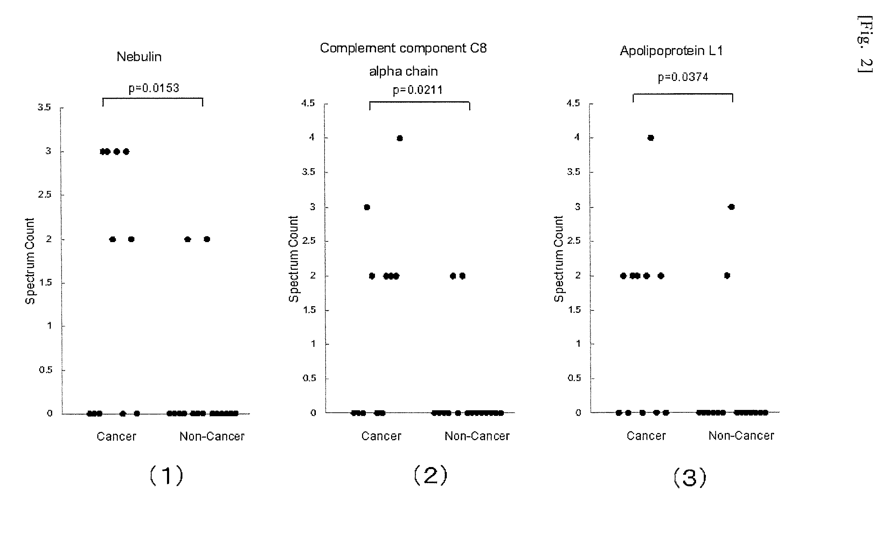

Fig. 2 shows the results of detection of each protein component in a sample derived from breast cancer patients and non-breast cancer subjects obtained in Example 1 and the statistical results obtained based on this result, wherein represents the results of measurement of (1) nebulin, (2) complement component C8 α chain, and (3) apolipoprotein L1.

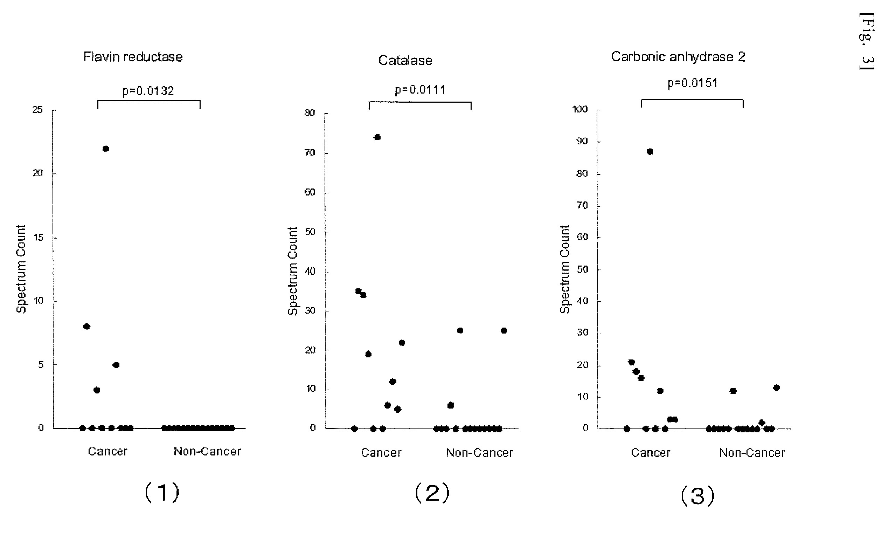

Fig. 3 shows the results of detection of each protein component in a sample derived from breast cancer patients and non-breast cancer subjects obtained in Example 1 and the statistical results obtained based on this result, wherein represents the results of measurement of (1) flavin reductase, (2) catalase, and (3) carbonic anhydrase 2.

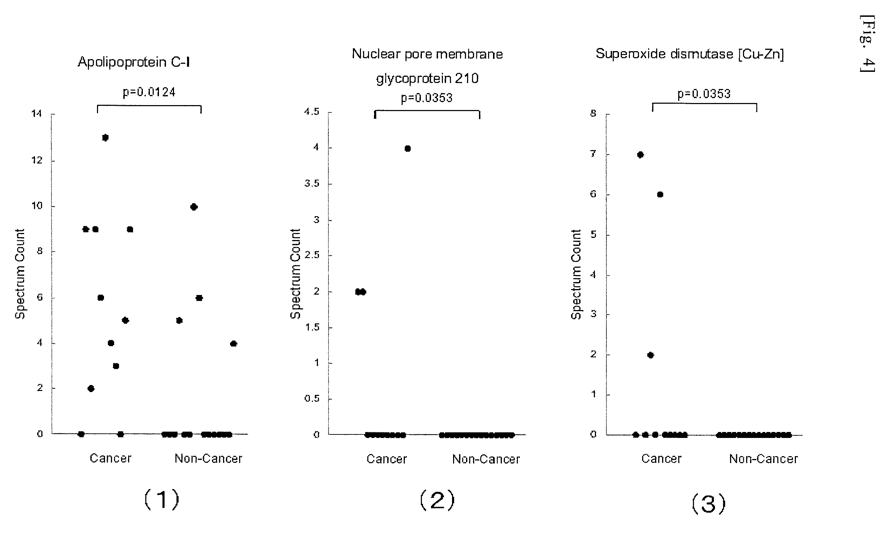

Fig. 4 shows the results of detection of each protein component in a sample derived from breast cancer patients and non-breast cancer subjects obtained in Example 1 and the statistical results obtained based on this result, wherein represents the results of measurement of (1) apolipoprotein C-I, (2) nuclear pore glycoprotein 210, and (3) superoxide dismutase [Cu-Zn].

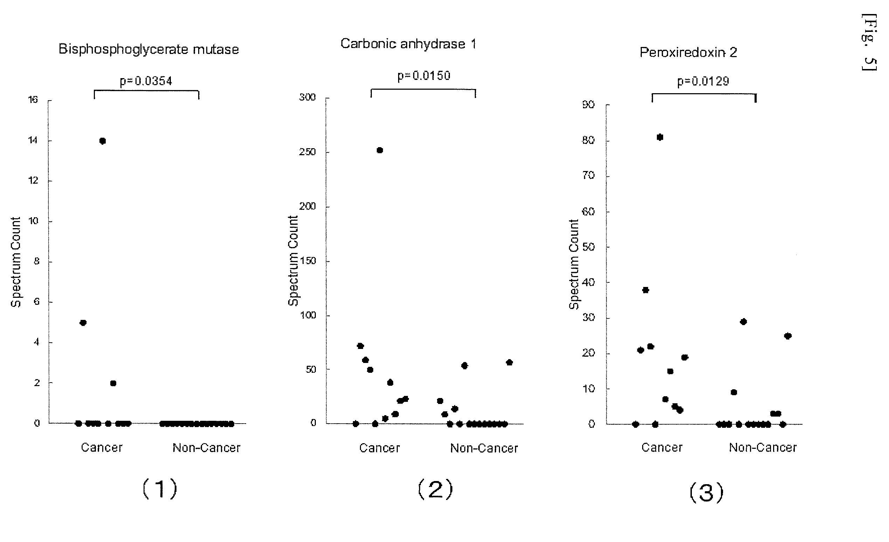

Fig. 5 shows the results of detection of each protein component in a sample derived from breast cancer patients and non-breast cancer subjects obtained in Example 1 and the statistical results obtained based on this result, wherein represents the results of measurement of (1) bisphosphoglycerate mutase, (2) carbonic anhydrase 1, and (3) peroxiredoxin-2.

### DESCRIPTION OF EMBODIMENTS

The breast cancer marker of the present invention includes a breast cancer marker selected from the group consisting of carboxypeptidase N subunit 2, extracellular matrix protein 1, serum amyloid P-component, nebulin, complement component C8 α chain, apolipoprotein L1, flavin reductase, catalase, carbonic anhydrase 2, apolipoprotein C-I, nuclear pore glycoprotein 210, superoxide dismutase [Cu-Zn], bisphosphoglycerate mutase, carbonic anhydrase 1, and peroxiredoxin-2 (hereinafter, these are sometimes referred to as "breast cancer marker of the present invention", collectively). These components themselves are already known, but the usefulness of them as an indicator (breast cancer marker) for determining breast cancer was revealed by the present inventors for the first time.

The method for determining breast cancer of the present invention is "A method for determining breast cancer comprising detecting the breast cancer marker(s) of the present invention, and determining breast cancer based on the results of the detection".

The method for detecting breast cancer marker of the present invention to be carried out for this purpose includes the conventional methods of detecting and measuring individual component of the breast cancer marker of the present invention. Reagents for detecting and measuring the breast cancer marker to be used therein may be any reagent to be used in the conventional methods for detecting and measuring each component of the aforementioned breast cancer marker.

An example of the method for detecting a breast cancer marker of the present invention includes the method according to the immunological measurement method well-known per se using, for example, a substance having an affinity for the breast cancer marker of the present invention (for example, antibodies, lectins, polysaccharides, DNA, enzyme substrates, proteins, various receptors, various ligands, etc.), such as so-called enzyme immunoassay (EIA), radioimmunoassay (RIA), enzyme-linked immunosorbent assay (ELISA), fluorescence immunoassay (FIA), a measurement method by simple immunochromatography; in addition to these methods, the methods by combination of these methods with high-performance liquid chromatography (HPLC), electrophoresis, capillary electrophoresis, capillary chip electrophoresis method, and the like are included. The principle of the measurement includes, for example, the sandwich method, a competitive method, double antibody method, etc., but it is not limited thereto.

In addition, the breast cancer marker of the present invention may be detected, for example, by a measurement method in accordance with immuno-agglutination method such as nephelometric immunoassay, turbidimetric immunoassay. These detection methods may also be carried out according to a method well-known per se.

An antibody against breast cancer marker to be used for detecting breast cancer marker of the present invention is not particularly limited as long as it is an antibody capable of recognizing the breast cancer marker of the present invention or a partial peptide thereof, or a salt thereof. For example, it may be either a polyclonal antibody or a monoclonal antibody, and it is arbitrary to use them alone or in appropriate combination, or the like. In addition, these antibodies are digested, if necessary, with an enzyme such as pepsin or papain, and may be used in the form of F(ab')₂, Fab', or Fab. Furthermore, commercially available antibodies against the breast cancer marker of the present invention can also be used.

The reagent and its concentration at the time of detection, and the measurement conditions and the like (reaction temperature, reaction time, pH at the reaction, measuring wavelength, measuring devices, etc.) on the occasion of performing the detection, which are used to detect the above-described breast cancer marker of the present invention, may all be set in accordance with the measurement procedure of the above described immunological assay well-known per se, and automatic analyzer or spectrophotometric system and the like, which are usually used in this field, can also be used without exception.

In these reagents to be used for detection of breast cancer marker of the present invention described above, the reagents which are commonly used in this field, for example, buffering agents, reaction accelerators, sugars, proteins, salts, stabilizers such as surfactant and preservatives, and at the same time, which do not inhibit the stability of coexisting reagents etc., and do not inhibit the reaction of the breast cancer marker of the present invention and an antibody etc. against the breast cancer marker, may be included. In addition, the concentration thereof may also be selected appropriately from the range of concentration commonly used in this field.

The method for detecting breast cancer marker, in the case where the breast cancer marker of detection target is an enzyme, includes a method well-known per se such as a method in which chromogenic reaction is introduced by reacting this with a coloring reagent, and the amount of resultant pigment is measured with a spectrophotometer. The coloring reagent and the like used for such purpose includes the coloring agents and the like commonly used in this field, and it is selected appropriately according to each enzyme of the measurement target. In addition, the concentration of use thereof may also be set appropriately from the range of concentrations commonly used in this field.

In addition, the breast cancer marker may also be detected by combination of two or more kinds of the breast cancer markers of the present invention. The combination is not particularly limited and two or more kinds from the breast cancer markers of the present invention may be combined arbitrarily.

Furthermore, the combination may be any combination of one or more kinds of breast cancer markers of the present invention with one or more kinds of breast cancer markers other than the breast cancer markers of the present invention.

It should be noted that, the detection of breast cancer marker of the present invention may be carried out by a measurement system, not being limited to the manual means, but using an automatic analyzer. The combination of the reagents in the case where the measurement is carried out by using the manual means or an automatic analyzer is not particularly limited, and may be used by selecting appropriately the combination of reagents assumed to be the best, according to the environment of the automatic analyzer and its model to be applied or taking other factors into consideration.

Furthermore, the breast cancer marker of the present invention may be detected by performing an comprehensive analysis of protein by so-called nano LC-ESI-MS/MS. The nano LC-ESI-MS/MS is a method in which a nano-liquid chromatography is employed as a separation means, and solution is sent at a low flow rate of several hundred nL/min, and after separating the components in a sample by the liquid chromatography as to a sample consisting of a large number of components, mass spectrometry by electrospray ionization method online is carried out, and then, as to mass components identified thereby, additional mass spectrometry using collision-induced dissociation is carried out once again. In this analytical method, after the mass analysis is carried out once and only particular mass component is subjected to mass spectrometry again and the obtained fragment pattern is further analyzed, therefore, a more detailed analysis can be carried out.

When the breast cancer marker of the present invention is detected by nano LC-ESI-MS/MS, for example, using two separation systems with two different pH for the above-described LC part of the nano LC-ESI-MS/MS, the detection may be carried out by performing comprehensive analysis of protein by two-dimensional nano LC-ESI-MS/MS.

A specific example of a method for detecting a breast cancer marker of the present invention by two-dimensional nano LC-ESI-MS/MS is as follows.

As the liquid chromatograph of the first dimension, for example, a reverse phase column is used, and a mobile phase is consisted of, for example, a basic solution (pH 9 to 10) such as about 20 mM aqueous ammonium formate solution or about 70 mM aqueous triethylamine solution and acetonitrile, a solution is sent at a low flow rate of a few µL/min (preferably, 5 to 500 µL/min), and the components in the sample are separated, and fractionated into about dozens of fractions, for example, at fixed time intervals from 1 to 10 minutes intervals. Then, after evaporating the solvent in each fraction fractionated, 0.1 % aqueous trifluoroacetic acid solution for each fraction was added to dissolve the separated components.

Then, the whole volume of each fractionated solution obtained is subjected to the liquid chromatography of the second dimension, and separated in the following way. That is, a whole volume of solution of the above-described each fraction is applied to a reverse-phase chromatography column, a mobile phase is consisted of, for example, an acidic solution (pH 2 to 3) such as 0.1% aqueous formic acid solution and acetonitrile/0.1% formic acid, a solution is sent at a low flow rate of several ten to one hundred nL/min (preferably, 50 to 500 nL/min), and a large number of components in each fraction are separated, and ESI-MS/MS is carried out online.

The analysis performed with a two-dimensional nano LC-ESI-MS/MS is more improved in the resolution than that performed with the nano LC-ESI-MS/MS single dimension, therefore, more comprehensive analysis of proteins becomes possible. By measuring with an internal standard substance and the like, the quantitative analysis is also possible.

By the method described above, the breast cancer marker of the present invention is detected, and based on results thereof, the data on the breast cancer marker of the present invention (for example, information on the presence or absence, the extent of the increase in the concentration and the amount of the breast cancer marker of the present invention) for determining a breast cancer using the breast cancer marker of the present invention as an indicator is obtained. Using the data obtained, for example, by the following method, determination (Diagnosis and test) of breast cancer is carried out.

That is, for example, detection of the breast cancer marker of the present invention in a sample derived from a subject is carried out, and from the test result stating that the aforementioned breast cancer marker is detected, it is determined that the subject might be at risk of breast cancer, or the subject is in high risk of breast cancer. In addition, in the case that the aforementioned breast cancer marker has not been detected, it is determined that there is no risk of breast cancer in the subject, or the risk of breast cancer is low, and the like.

As other methods, at first, the amount of a breast cancer marker of the present invention contained in a sample is determined by the above described method. Then the obtained result is compared with the results obtained by the same manner using a sample derived from non-breast cancer subject as a separate sample in advance, and from the test result stating that the amount of the aforementioned breast cancer marker contained in a sample derived from the subject is greater than that contained in a sample derived from non-breast cancer subject, it is possible to determine that the subject might be at risk of breast cancer, or the subject is in high risk of breast cancer. In addition, from the test results stating that a significant difference is not observed between the amount of the aforementioned breast cancer marker contained in the sample derived from the subject and the amount contained in the sample derived from non-breast cancer subject, it is possible to determine that there is no risk of breast cancer in the subject, or the risk of breast cancer is low.

In addition, a reference value is set in advance, and from the test results stating that the amount of breast cancer marker of the present invention from a subject is more than the reference value of the breast cancer marker, it is possible to determine that there is at risk of breast cancer in the subject, or the risk of breast cancer is high in the subject, and the like. In addition, setting plural judgment criteria corresponding to the amount of the aforementioned breast cancer marker or the quantitative range thereof [for example, (1) no risk of breast cancer, (2) low risk of breast cancer, (3) sign of breast cancer, (4) high risk of breast cancer, etc.], it is also possible to determine that into which judgment criteria the test result is applicable.

Furthermore, in the same subject, by comparing the amount of breast cancer marker of the present invention in a sample derived from the subject measured at some time point with the amount of the aforementioned breast cancer marker at different time points, and by evaluating the presence or absence of increase or decrease, and/or the degree of increase or decrease of the aforementioned breast cancer marker, diagnosis of progression or malignancy of breast cancer, or prognosis after surgery is possible. That is, from the test results stating that an increase in the amount of the aforementioned breast cancer marker is observed, a determination that the pathological condition has progressed to breast cancer (or malignancy of breast cancer has increased), or a sign of pathological progression to breast cancer is observed (or a sign of malignancy increase of breast cancer is observed), can be made. In addition, from the test results stating that an decrease in the amount of the aforementioned breast cancer marker in a sample derived from the subject was observed, a determination that the pathological condition of breast cancer was improved, or a sign of improvement of pathological condition is observed, can be made. In addition, from the test result stating that variation in the amount of the aforementioned breast cancer marker is not observed, a determination that there is no change in the pathological condition can be made.

Furthermore, a method in which two or more of breast cancer markers of the present invention are detected and measured, and determination of breast cancer is made on the basis of the results, is also included. For example, by detecting breast cancer marker of the present invention alone, or two or more in combination, the presence pattern of breast cancer marker(s) of the present invention characteristic of the breast cancer patients is obtained. Therefore, by detecting and identifying breast cancer marker(s) of the present invention from a sample of the nipple discharge, etc. from the subject, and by analyzing the presence pattern of these plural breast cancer markers of the present invention, the estimation of whether the subject is developing breast cancer is possible.

The combination of two or more of breast cancer markers may be selected arbitrarily from the group consisting of breast cancer markers of the present invention.

In addition, one or more of breast cancer markers of the present invention and one or more of breast cancer markers other than the breast cancer markers of the present invention are detected and measured, and determination of breast cancer may be carried out in the same manner as described above. Detection of breast cancer marker(s) other than the breast cancer markers of the present invention may be carried out in a manner well-known per se in accordance with the respective breast cancer markers.

The sample to be used for detecting breast cancer marker of the present invention includes, nipple discharge from a subject, or a sample derived from a living organism such as blood (whole blood), serum, plasma, urine, and lymph from a subject, but it is not limited thereto.

The nipple discharge includes the nipple discharge secreted naturally from breast of one or both sides of the subject, secretory fluid exuded by massage of the breast, and nipple aspirate fluid, or the like, but it is not limited thereto.

If the nipple discharge is used as a sample, the sample can be taken non-invasively from the subject, and it is excellent in the terms that burden on the patient is much less.

"A kit for detecting a breast cancer marker in order to perform the determination of breast cancer" of the present invention is the one which comprises the reagents for detecting breast cancer marker of the present invention as a constituent.

Preferred embodiment and specific example of "the reagent for detecting breast cancer marker of the present invention" and the constituent thereof is as described above in the description on the method for detecting breast cancer marker of the present invention. In addition, a preferred embodiment of the concentration, etc. of these reagents may be selected appropriately from the range of concentrations commonly used in this field.

For example, examples of the reagents for detecting the breast cancer marker of the present invention include those comprising a substance having affinity for the aforementioned breast cancer marker. In addition, example of the substance having affinity for the breast cancer marker thereof includes, for example, antibody or lectin, and the like.

The aforementioned kit may be either the one which comprises reagents or a kit for detecting one kind of breast cancer marker as the constituent, or the one which is comprised of a combination of plural reagents or kits for detecting plural breast cancer markers. In addition, as a combination of plural reagents or kits for detecting plural breast cancer markers, the combination of plural reagents or kits for detecting plural breast cancer markers of the present invention, or a combination of "one kind or plural reagents or one kind or plural kits for detecting one kind or plural breast cancer markers of the present invention" and "one kind or plural reagents or one kind or plural kits for detecting one kind or plural breast cancer markers other than the breast cancer markers of the present invention", and the like are included.

In addition, as to the reagent contained in these kits, there may be included reagents which are commonly used in this field, for example, buffering agents, reaction accelerators, sugars, proteins, salts, stabilizers such as surfactant and preservatives, which neither inhibit the stability of coexisting reagents etc., nor inhibit the reaction of the breast cancer marker of the present invention and an antibody (or lectin, etc.). In addition, the concentration thereof may also be selected appropriately from the range of concentration commonly used in this field.

Furthermore, it may be a kit which is made by combining the standard of breast cancer marker(s) for preparing a standard curve to be used for detecting the aforementioned breast cancer marker(s). For the aforementioned standards, the commercially available standards or those produced according to known methods, may also be used.

Hereinafter, the present invention is explained specifically based on examples, but the scope of the present invention should not be limited thereto.

### Examples

### Example 1: Selection of breast cancer marker

### (1) Pretreatment of sample

Nipple discharge (a few µL) taken from each breast cancer patients (11 persons) was added to 100 µL of sample stock solution (an aqueous solution containing 50 mM ammonium hydrogencarbonate and 0.02% sodium azide), and centrifuged at 18,000 x g, for 5 minutes at 4°C. After separating the supernatant, protein concentration was measured by ultraviolet absorption spectrometry using an ultraviolet ray of 280 nm wavelength, and the protein concentration was adjusted so as to provide 0.5 mg/mL using 50 mM aqueous ammonium hydrogencarbonate solution. After that, 1.41 µL of 45 mM aqueous dithiothreitol solution was added to reduce the solution, and trifluoroethanol was added thereto to denature the protein so that it provides 50%, and heated at 60°C for 1 hour. After heating, the solution was brought back to room temperature, and 1.41 µL of 100 mM aqueous iodoacetamide solution was added, and the solution was left under a dark environment at room temperature for 1 hour to perform alkylation. Then, 10 µL of aliquot was separated from the alkylation products, and 90 µL of 50 mM aqueous ammonium hydrogencarbonate solution was added to adjust so that the percentage of trifluoroethanol becomes 5%. Furthermore, 1.2 µL of 100 ng/µL trypsin solution (mass spectrometry grade, Promega Corporation, Madison, WI, USA) (trypsin content: 1/20 amount of the protein weight) was added, and the solution was incubated at 37°C overnight. The tryptic digest product obtained (approximately 350 fmol/µL in terms of protein) was subjected to two-dimensional nano LC-ESI-MS/MS measurement by the following method.

Separately, the nipple discharge collected from medical examinee (subject) who complained that secretory fluid was secreted from a nipple, as indefinite complaint, and who were diagnosed as not the breast cancer (15 persons), were treated in the same manner as described above.

### (2) Two-dimensional nano LC-ESI-MS/MS measurement

Using two-dimensional Nano HPLC system (UltiMate 3000; Thermo Fisher Scientific Inc. (Dionex), Waltham, MA, USA) equipped with Acclaim PA2, 300 µm i.d. x 15 cm reverse-phase column (Thermo Fisher Scientific Inc. (Dionex), Waltham, MA, USA), a 40 µL of aliquot of the trypsin digestion product obtained in above (1) was separated by gradient elution using solvent A : 20 mM aqueous ammonium formate solution, pH 10.0 and solvent B : acetonitrile for 75 minutes (solvent B of 12 to 95%), and 26 fractions were obtained. The solvent in those fractions was evaporated by heating at 70°C for 4 hours. After that, 30 µL of 0.1% aqueous trifluoroacetic acid solution was added to each fraction to dissolve the separated components. Subsequently, using a L-column Micro L2-C18, 75 µm i.d. x 15 cm reverse phase column (produced by Chemicals Evaluation and Research Institute, Japan), each of the whole volume of each fraction solution obtained was separated by gradient elution using solvent A : 0.1% aqueous formic acid solution and solvent B : acetonitrile/0.1% formic acid for 45 minutes (2 to 95% of solvent B), and analyzed sequentially online using data dependent scan by ESI-ion trap mass spectrometer (HCTultra; Bruker Daltonik GmbH, Bremen, Germany) equipped with nano-ESI ion source. For each sample, runs were carried out twice.

Each condition in the two-dimensional nano LC-ESI-MS/MS analysis is as follows.

### i) LC condition

### (First dimension)

HPLC column: Acclaim PA2, 300 µm i.d. x 15 cm reverse phase column (produced by Thermo Fisher Scientific Inc. (Dionex))
Flow rate: 6.0 µL/min
Solvent A: 20 mM aqueous ammonium formate solution, pH 10.0
Solvent B: Acetonitrile
Elution: 0 minute → 19 minutes: Linear gradient from 12% to 20% solvent B,
   19 minutes → 29 minutes: Linear gradient to 48% solvent B,
   29 minutes → 34 minutes: Washing with 95% solvent B,
   34 minutes → 75 minutes: Equilibration with 2% solvent B.
   (It should be noted that, since the concentrations of solvent B was raised immediately after the start of elution, the concentration of solvent B in the eluent has become 12% in almost 0 minutes after the start of elution.)
Column temperature: 35°C
Injection volume: 40 µL
Number of fractions: 26

### (Second dimension)

HPLC column: L-column Micro L2-C18, 75 µm i.d. x 15 cm reverse phase column (produced by Chemicals Evaluation and Research Institute, Japan)
Flow rate: 300 nL/min
Solvent A: 0.1% aqueous formic acid solution, pH 2.0
Solvent B: Acetonitrile/0.1% formic acid
Elution: 0 minutes → 1 minute: Linear gradient to 8% solvent B
   1 minute → 20 minutes: Linear gradient to 20% solvent B
   20 minutes → 25 minutes: Linear gradient to 35% solvent B
   25 minutes → 30 minutes: Washing with 95% solvent B
   30 minutes → 45 minutes: Equilibration with 2% solvent B.
Column temperature: Room temperature
Injection volume: 30 µL

### ii) ESI-MS/MS condition

Ionization method: Electrospray ionization method, ESI method
Measured ion: Positive ion
Spray voltage: 1200 V
Dry gas: Nitrogen (4 L/min)
Ion source temperature: 160°C
Scanning range: *m*/*z* 300 to 1,500 (at MS/MS measurement: *m*/*z* 50 to 2,200)
Precursor ion selection number: 4 ions/mass spectrum
Minimum precursor ion intensity: 50,000 counts
Precursor ion selective exclusion time: 30 s
Precursor ion exclusion valence: 1

### iii) Protein identification analysis condition

Protein search engine: Mascot (Matrix Science Ltd., London, UK)
Protein search technique: MS/MS Ion Search
Protein database: Swiss-Prot
Animal species classification at protein search: Homo sapiens (human)
Post-translational modification setting at protein search: Carbamidomethyl (cysteine residue modification)
Trypsin non-cleavage site allowable number of times: 1
Precursor ion mass tolerance: ± 0.5 Da
Product ion mass tolerance: ± 1 Da
Identified protein analysis software: Scaffold (Proteome Software, Inc., Portland, OR, USA)

### (3) Results of two-dimensional nano LC-ESI-MS/MS measurement

By two-dimensional nano LC-ESI-MS/MS measurement, 350 species of protein components from samples derived from breast cancer patients, 479 species of protein components from samples derived from non-breast cancer subjects, a total of 575 species of protein components were detected. In this analysis, it should be noted that peroxiredoxin-1 described in Patent Literature 2 was detected in both breast cancer patients sample and non-breast cancer subjects sample, and further, it was not detection specific to breast cancer patient.

Subsequently, in the following way, proteins detected specifically from a breast cancer patient samples were selected as breast cancer marker candidates.

### (4) Screening of breast cancer marker candidate

Based on the results of the above-described (3),
(i) Rate of number of breast cancer patients (n1) in whom each protein component was detected in a sample, among 11 persons of breast cancer patients examined (ratio A),
(ii) Rate of number of non-breast cancer subjects (n2) in whom each protein component was detected in a sample, among 15 persons of non-breast cancer subjects examined (ratio B), and
(iii) Ratio A/ratio B (ratio C)
were calculated.

For want of space, among 575 of protein components detected in the above (3), a list of the protein components in which ratio C was 2.00 or more is shown in Table 1 below.

**Table 1**

| Detected component | Detected component |
|---|---|
| Carboxypeptidase N subunit 2 | Disks large homolog 5 |
| Extracellular matrix protein 1 | Selenium-binding protein 1 |
| Serum amyloid P-component | Complement component C8 gamma chain |
| Nebulin | Mucin-5B |
| Complement component C8 alpha chain | Complement Clq subcomponent subunit C |
| Apolipoprotein L1 | Keratin, type II cytoskeletal 5 |
| Secretoglobin family 1D member 2 | Carbonic anhydrase 1 |
| 14-3-3 protein beta/alpha | Peroxiredoxin-2 |
| Keratin, type II cytoskeletal 2 epidermal | Apolipoprotein A-IV |
| Flavin reductase | Centriolin |
| Catalase | Plasma kallikrein |
| Apolipoprotein C-III | Attractin |
| Hemoglobin subunit gamma-2 | Keratin, type II cytoskeletal 6B |
| Ig heavy chain V-III region WEA | Peptidyl-prolyl cis-trans isomerase A |
| Peroxiredoxin-6 | Keratin, type I cytoskeletal 19 |
| Carbonic anhydrase 2 | Msx2-interacting protein |
| Apolipoprotein C-I | WD repeat-containing protein 87 |
| Ig lambda chain V-III region SH | Spectrin alpha chain, erythrocyte |
| Nuclear pore membrane glycoprotein 210 | DNA/RNA-binding protein KIN17 |
| Superoxide dismutase [Cu-Zn] | Ig heavy chain V-II region WAH |
| Bisphosphoglycerate mutase | Ig kappa chain V-I region Mev |
| Obscurin | Keratin, type I cytoskeletal 18 |
| Hemoglobin subunit delta | Inter-alpha-trypsin inhibitor heavy chain H3 |
| Complement C5 | Ficolin-3 |
| Fibrous sheath-interacting protein 2 | Ig delta chain C region |
| Desmoplakin | Delta-aminolevulinic acid dehydratase |
| Centromere protein F | Acylamino-acid-releasing enzyme |
| Aminopeptidase N | |

And, the protein components etc., which were suspected of blood-derived component, were excluded from the list of the protein components listed in Table 1, and the protein component in which the ratio C is 2.4 or more, and at the same time, a statistically significant difference was observed between breast cancer patient and non-breast cancer subject in a spectrum count value indicating the abundance of the protein component calculated based on the peptide peaks detected in the mass spectrum (the protein component of p < 0.05 in the Mann-Whitney U-test), was selected as a breast cancer marker candidate. The ratio A, B, and C of the selected breast cancer marker candidate and each breast cancer marker candidate are shown in Table 2, and the statistical results were shown in Fig. 1 to Fig. 5.

It should be noted that, this means that, in Table 2, if the ratio B was 0, that breast cancer marker was not detected from non-breast cancer subject, resulting in that it is impossible to calculate the value of the ratio C. Therefore, the ratio C in this case, the same value as the number of breast cancer patients of n1 was indicated (*).

**Table 2**

| | Breast cancer patient | | Non-breast cancer subject | | RatioA/ Ratio B |
|---|---|---|---|---|---|
| | n1 | Ratio A | n2 | Ratio B | Ratio C |
| Carboxypeptidase N subunit 2 | 5 | 0.455 | 1 | 0.067 | 6.791 |
| Extracellular matrix protein 1 | 5 | 0.455 | 0 | 0.000 | 5.000* |
| Serum amyloid P-component | 7 | 0.636 | 2 | 0.133 | 4.782 |
| Nebulin | 6 | 0.545 | 2 | 0.133 | 4.098 |
| Complement component C8 alpha chain | 6 | 0.545 | 2 | 0.133 | 4.098 |
| Apolipoprotein L1 | 6 | 0.545 | 2 | 0.133 | 4.098 |
| Flavin reductase | 4 | 0.364 | 0 | 0.000 | 4.000* |
| Catalase | 8 | 0.727 | 3 | 0.200 | 3.635 |
| Carbonic anhydrase 2 | 7 | 0.636 | 3 | 0.200 | 3.180 |
| Apolipoprotein C-I | 9 | 0.818 | 4 | 0.267 | 3.064 |
| Nuclear pore membrane glycoprotein 210 | 3 | 0.273 | 0 | 0.000 | 3.000* |
| Superoxide dismutase [Cu-Zn] | 3 | 0.273 | 0 | 0.000 | 3.000* |
| Bisphosphoglycerate mutase | 3 | 0.273 | 0 | 0.000 | 3.000* |
| Carbonic anhydrase 1 | 9 | 0.818 | 5 | 0.333 | 2.456 |
| Peroxiredoxin-2 | 9 | 0.818 | 5 | 0.333 | 2.456 |

That is, as listed in Table 2, Carboxypeptidase N subunit 2, Extracellular matrix protein 1, Serum amyloid P-component, Nebulin, Complement component C8 alpha chain, Apolipoprotein L1, Flavin reductase, Catalase, Carbonic anhydrase 2, Apolipoprotein C-I, Nuclear pore membrane glycoprotein 210, Superoxide dismutase [Cu-Zn], Bisphosphoglycerate mutase, Carbonic anhydrase 1, and Peroxiredoxin-2 were selected as a breast cancer marker of the present invention.

As is clear from Table 2 and Figs. 1 to 5, these breast cancer markers were present in high concentrations in the nipple discharge of breast cancer patients as compared with concentration in the nipple discharge of non-breast cancer subjects. In addition, since there is no track record of these components that was used as a breast cancer marker, these are very promising as novel breast cancer markers.

In addition, it is expected that the determination of breast cancer and the progression thereof can be carried out by detecting these breast cancer markers alone, or two kinds or more markers in combination. For example, by detecting these breast cancer makers alone, or two kinds or more markers in combination, the present pattern of the breast cancer markers of the present invention characteristic of the breast cancer patients is obtained. Therefore, it is expected that, by detecting and identifying a breast cancer marker of the present invention from a sample of the nipple discharge, etc. of the subject, and by analyzing the presence pattern of these plural breast cancer markers, the estimation of whether the subject has developed breast cancer will be possible.

### INDUSTRIAL APPLICABILITY

The determination method using the breast cancer marker of the present invention enables the determination (diagnosis, test) of breast cancer with higher accuracy.

In addition, since the breast cancer marker(s) of the present invention are found, for example, in nipple discharge, it is possible to perform determination (diagnosis, test) of breast cancer non-invasively and simply. As a result, the breast cancer diagnosis according to the present invention in which mental and physical distress is small is expected to encourage the younger generation and the young parous and multiparous women during or after lactation to perform breast cancer screening on a voluntary basis, and expected to improve the breast cancer screening rate in Japan which is lower as compared with that in the world, and to provide the economic effect due to the reduction of breast cancer mortality.

## Claims

1. A breast cancer marker selected from the group consisting of carboxypeptidase N subunit 2, extracellular matrix protein 1, serum amyloid P-component, nebulin, complement component C8 α chain, apolipoprotein L1, flavin reductase, catalase, carbonic anhydrase 2, apolipoprotein C-I, nuclear pore glycoprotein 210, superoxide dismutase [Cu-Zn], bisphosphoglycerate mutase, carbonic anhydrase 1, and peroxiredoxin-2.

2. A method for determining breast cancer, comprising:
detecting one or more of breast cancer markers selected optionally from the group consisting of carboxypeptidase N subunit 2, extracellular matrix protein 1, serum amyloid P-component, nebulin, complement component C8 α chain, apolipoprotein L1, flavin reductase, catalase,
carbonic anhydrase 2, apolipoprotein C-I, nuclear pore glycoprotein 210, superoxide dismutase [Cu-Zn], bisphosphoglycerate mutase, carbonic anhydrase 1, and peroxiredoxin-2 in a sample, and
determining breast cancer on the basis of the results of the detection.

3. The method according to claim 2, wherein the sample is nipple discharge.

4. The method according to claim 2, wherein the sample is serum, plasma, or whole blood.

5. A kit for detecting a breast cancer marker to determine breast cancer comprising a reagent for detecting breast cancer marker selected from the group consisting of carboxypeptidase N subunit 2, extracellular matrix protein 1, serum amyloid P-component, nebulin, complement component C8 α chain, apolipoprotein L1, flavin reductase, catalase, carbonic anhydrase 2, apolipoprotein C-I, nuclear pore glycoprotein 210, superoxide dismutase [Cu-Zn], bisphosphoglycerate mutase, carbonic anhydrase 1, and peroxiredoxin-2.

6. The kit according to claim 5, wherein the reagent comprises a substance having an affinity for the breast cancer marker.

7. The kit according to claim 6, wherein the substance having an affinity for the breast cancer marker is an antibody.
